# EUROPEAN PATENT SPECIFICATION

(11) **EP 1 399 066 B1**
(45) Date of publication and mention of the grant of the patent: **25.10.2006**
(21) Application number: 02744255.7
(22) Date of filing: 07.06.2002
(51) Int. Cl.: A61B 5/15, A61B 5/145

(54) **DEVICES FOR THE EXPRESSION OF BODILY FLUIDS FROM AN INCISION**
VORRICHTUNGEN ZUM AUSDRÜCKEN VON KÖRPERFLÜSSIGKEITEN AUS EINEM EINSCHNITT
DISPOSITIFS D'EXPRESSION DE FLUIDES CORPORELS PROVENANT D'UNE INCISION

(30) Priority: 08.06.2001 US 296949 P; 08.06.2001 US 296950 P; 14.06.2001 US 879991; 29.08.2001 US 315873 P; 29.08.2001 US 315968 P
(43) Date of publication of application: 24.03.2004
(73) Proprietor: Roche Diagnostics GmbH, 68305 Mannheim (DE); F.HOFFMANN-LA ROCHE AG, 4070 Basel (CH)
(72) Inventor: PEREZ, Edward P., Menlo Park, CA 94025 (US); RANEY, Charles, C., Scotts Valley, CA 95066 (US); PATEL, Paul, Sunnyvale, CA 94086 (US)
(74) Representative: Jung, Michael
(86) International application number: PCT/US2002/018161
(87) International publication number: WO 2002/100276

(56) References cited:
- WO-A-97/42882
- WO-A-97/43962
- US-A- 3 741 197

## Description

### Background of the Invention

### Field of the Invention

The present invention relates to devices for expressing and sampling of a bodily fluid obtained from an incision in the skin, and more particularly to acquiring the fluid by capillary action. The invention also may include the combination of such sampling devices with incising, expressing, and/or testing systems.

### Description of the Prior Art

The acquisition and testing of bodily fluids is useful for many purposes, and continues to grow in importance for use in medical diagnosis and treatment, and in other diverse applications. In the medical field, it is desirable for lay operators to perform tests routinely, quickly and reproducibly outside of a laboratory setting, with rapid results and a readout of the resulting test information. Testing can be performed on various bodily fluids, and for certain applications is particularly related to the testing of blood and/or interstitial fluid. Such fluids can be tested for a variety of characteristics of the fluid, or analytes contained in the fluid, in order to identify a medical condition, determine therapeutic responses, assess the progress of treatment, and the like.

The testing of bodily fluids basically involves the steps of obtaining the fluid sample, transferring the sample to a test device, conducting a test on the fluid sample, and displaying the results. These steps are generally performed by a plurality of separate instruments or devices.

One method of acquiring the fluid sample involves inserting a hollow needle or syringe into a vein or artery in order to withdraw a blood sample. However, such direct vascular blood sampling can have several limitations, including pain, infection, and hematoma and other bleeding complications. In addition, direct vascular blood sampling is not suitable for repeating on a routine basis, can be extremely difficult and is not advised for patients to perform on themselves.

The other common technique for collecting a bodily fluid sample is to form an incision in the skin to bring the fluid to the skin surface. A lancet, knife or other cutting instrument is used to form the incision in the skin. The resulting blood or interstitial fluid specimen is then collected in a small tube or other container, or is placed directly in contact with a test strip. The fingertip is frequently used as the fluid source because it is highly vascularized and therefore produces a good quantity of blood. However, the fingertip also has a large concentration of nerve endings, and lancing the fingertip can therefore be painful. Alternate sampling sites, such as the palm of the hand, forearm, earlobe and the like, may be useful for sampling, and are less painful. However, they also produce lesser amounts of blood. These alternate sites therefore are generally appropriate for use only for test systems requiring relatively small amounts of fluid, or if steps are taken to facilitate the expression of the bodily fluid from the incision site.

Various methods and systems for incising the skin are known in the art. Exemplary lancing devices are shown, for example, in United States Patent Nos. Re 35,803, issued to Lange, et al. on May 19, 1998.; 4,924,879, issued to O'Brien on May 15, 1990; 5,879,311, issued to Duchon et al. on February 16, 1999; 5,857,983, issued to Douglas on January 12, 1999; 6,183,489, issued to Douglas et al. on February 6, 2001; 6,332,871, issued to Douglas et al. on December 25, 2001; and 5,964,718, issued to Duchon et al. on October 12, 1999. A representative commercial lancing device is the Accu-Chek Softclix lancet.

Document WO-A-97/43 962 discloses a device for expressing bodily fluid from an incision in the skin comprising a body with a cavity connected to an expression member which is movable between an outer position and an inner position whereby the skin-engaging surface of the member draws the skin into the member.

Patients are frequently advised to urge fluid to the incision site, such as by applying pressure to the area surrounding the incision to milk or pump the fluid from the incision. Mechanical devices are also known to facilitate the expression of bodily fluid from an incision. Such devices are shown, for example, in United States Patent Nos. 5,879,311, issued to Duchon et al. on February 16, 1999; 5,857,983, issued to Douglas on January 12, 1999; 6,183,489, issued to Douglas et al. on February 6, 2001; 5,951,492, issued to Douglas et al. on September 14, 1999; 5,951,493, issued to Douglas et al. on September 14, 1999; 5,964,718, issued to Duchon et al. on October 12, 1999; and 6,086,545, issued to Roe et al. on July 11, 2000. A representative commercial product that promotes the expression of bodily fluid from an incision is the Amira AtLast blood glucose system.

The acquisition of the produced bodily fluid, hereafter referred to as the "sampling" of the fluid, can take various forms. Once the fluid specimen comes to the skin surface at the incision, a sampling device is placed into contact with the fluid. Such devices may include, for example, systems in which a tube or test strip is either located adjacent the incision site prior to forming the incision, or is moved to the incision site shortly after the incision has been formed. A sampling tube may acquire the fluid by suction or by capillary action. Such sampling systems may include, for example, the systems shown in US Patent Nos. 6,048,352, issued to Douglas et al. on April 11, 2000; 6,099,484, issued to Douglas et al. on August 8, 2000; and 6,332,871, issued to Douglas et al. on December 25, 2001. Examples of commercial sampling devices include the Roche Compact, Amira AtLast, Glucometer Elite and Therasense FreeStyle test strips.

The bodily fluid sample may be analyzed for a variety of properties or components, as is well known in the art. For example, such analysis may be directed to hematocrit, blood glucose, coagulation, lead, iron, etc. Testing systems include such means as optical (e.g., reflectance, absorption, fluorescence, Raman, etc.), electrochemical; and magnetic means for analyzing the sampled fluid. Examples of such test systems include those in US Patent Nos. 5,824,491, issued to Priest et al. on October 20, 1998; 5,962,215, issued to Douglas et al. on October 5, 1999; and 5,776,719, issued to Douglas et al. on July 7, 1998.

Typically, a test system takes advantage of a reaction between the bodily fluid to be tested and a reagent present in the test system. For example, an optical test strip will generally rely upon a color change, i.e., a change in the wavelength absorbed or reflected by dye formed by the reagent system used. See, e.g., US Patent Nos. 3,802,842; 4,061,468; and 4,490,465.

A common medical test is the measurement of blood glucose level. The glucose level can be determined directly by analysis of the blood, or indirectly by analysis of other fluids such as interstitial fluid. Diabetics are generally instructed to measure their blood glucose level several times a day, depending on the nature and severity of their diabetes. Based upon the observed pattern in the measured glucose levels, the patient and physician determine the appropriate level of insulin to be administered, also taking into account such issues as diet, exercise and other factors.

In testing for the presence of an analyte such as glucose in a bodily fluid, test systems are commonly used which take advantage of an oxidation/reduction reaction which occurs using an oxidase/peroxidase detection chemistry. The test reagent is exposed to a sample of the bodily fluid for a suitable period of time, and there is a color change if the analyte (glucose) is present. Typically, the intensity of this change is proportional to the concentration of analyte in the sample. The color of the reagent is then compared to a known standard which enables one to determine the amount of analyte present in the sample. This determination can be made, for example, by a visual check or by an instrument, such as a reflectance spectrophotometer at a selected wavelength, or a blood glucose meter. Electrochemical and other systems are also well known for testing bodily fluids for properties on constituents.

The present invention provides for enhancing the fluid sampling and testing by assisting in the expression of the fluid from the incision. Expression of the fluid is always useful in order to increase the quantity of bodily fluid available for acquisition by a sampling device. Such larger quantities make it easier to quickly and reliably acquire the fluid, and reduce the potential that there will be an insufficient quantity of fluid acquired for testing to be performed. When used at the fingertip, expression results in an even larger quantity of fluid being produced in a shorter period of time, thereby providing a suitable amount of fluid for tests requiring relatively larger quantities. When used at alternate sites, the fluid expression can be important to provide a sufficient quantity of fluid to be acquired and tested by a given system.

### Summary of the Invention

The present invention provides devices for the expression of bodily fluid from an incision in the skin. The invention is defined in claim 1.

In accordance with the present invention, there is provided a device for expressing bodily fluid from an incision site including a body defining a cavity and a bi-stable expression member connected to the body adjacent to the interior cavity. The expression member includes a skin-engaging portion having first and second stable conditions, with the portion in the first stable condition being outwardly bulged relative to the body, and the portion in the second stable condition being inverted and received within the cavity of the body. The exterior of the skin-engaging portion includes an exterior surface and defines an aperture communicating between the exterior surface and the cavity. The skin-engaging portion engages the skin and draws the skin into the member as the skin-engaging portion inverts from the first condition to the second condition. The exterior surface of the skin-engaging portion is optionally configured or treated to enhance the grasping of the skin, such as by providing projections or a sticky coating.

### Brief Description of the Drawings

FIG. 1 is a side, elevational view of an embodiment of an expression system in accordance with the present invention.
FIG. 2 is a side, cross-sectional view of the device of FIG. 1.
FIG. 3 is a side, cross-sectional view of the device of FIG. 1, showing the expression member in the inverted position.

### Description of the Preferred Embodiment

For the purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further modifications in the illustrated devices and methods, and such further applications of the principles of the invention as illustrated therein being contemplated as would normally occur to one skilled in the art to which the invention relates.

The present invention provides a variety of devices which are useful in enhancing the expression of fluid from an incision in the skin. This expression of the fluid results in a fluid sample that is larger, and/or produced more quickly, than would otherwise result. Expression of a bodily fluid from an incision is desirable in many instances. The lancing of the skin may produce a sufficient quantity of blood or interstitial fluid, but expression of the fluid from the incision will provide greater assurance that a sufficient quantity has been produced, and that the sample may be successfully and readily acquired. Also, expression of the bodily fluid is advantageous in the use of alternate test sites, such as the palm or forearm, because the quantity of fluid produced without expression can be significantly less than the amount produced at the fingertip. While some test devices will operate with greatly reduced fluid volumes, a greater volume of fluid makes it easier to acquire the fluid for testing. The present invention provides devices that greatly enhance the expression of fluid from an incision.

The expression of fluid is also important when used in combination devices. The expression of fluid in the context of an integrated lancing and sampling device, for example, is advantageous since it is more difficult to view the fluid sample and it is therefore more important that the sample is sufficient for sampling and testing purposes. The present invention is readily combined in a single, integrated unit with incising, sampling and/or testing devices.

The fluid is obtained from an incision formed in the surface of the skin. The incising of the skin may be accomplished by any suitable means, including cutting with a mechanical instrument, laser, high speed fluid stream, etc. Of these, lancing the skin is most common and is preferred, and specific descriptions herein use lancing for purposes of example. It will be appreciated, however, that lancing is only exemplary, and all forms of making an incision in the skin are included.

The depth of penetration generally controls the fluid produced, particularly in combination with the characteristics of the incision site. The present invention is useful with various bodily fluids, including blood or interstitial fluid. The incising device may be configured for production of either blood or interstitial fluid, for example, by controlling the distance which the incising device extends into the user's skin. For example, a depth of 0.25 mm to 4 mm will typically produce blood from the dermis, while a depth of 0.05 mm to 0.5 mm will produce interstitial fluid from the epidermis.

The concept that bodily fluid can be expressed from an incision by a variety of methods and devices that retain the fluid adjacent the incision and/or urge the fluid toward the incision. One concept involves constricting the area surrounding the incision, thereby retaining the bodily fluid within the constricted location and at the same time urging the fluid toward the incision. A second concept involves pressing the skin surrounding the incision, thereby increasing the pressure on the fluid and forcing it to move toward and out of the incision. A third concept involves the "kneading" of the skin by moving a device along the skin in the direction of the incision, thereby pushing the fluid toward and out of the incision.

The fluid expression functions to facilitate the production of fluid at the site of an incision. As used herein, the term "incision" is intended to cover any opening in the skin that permits direct access to the bodily fluid. Unless indicated otherwise, the expression systems can be used before and/or after the incision is formed. Therefore the term "incision site" is intended to include the site where an incision either has been or will be formed, unless from the context or express language it is clear otherwise.

One of the approaches to expression in accordance with the present invention is the constriction of the skin surrounding the site of the incision. The constriction may occur before, during and/or after the incision is formed. The term constriction is intended herein to refer to contacting the skin at locations outward of the incision site and then moving a portion of the skin inward toward the incision site and holding the skin in that position. The initial engagement of the skin and the subsequent movement inwardly of the skin essentially grasps the skin at the surface and pinches the skin in a manner to retain it in the constricted position. This provides a portion of skin around the incision site that is engaged by the constriction device and is retained in this position for a period of time, typically while the incising and sampling take place. The skin will have the appearance of a raised pucker or pinch of skin which includes the incision site. This constriction of the skin is distinguished from mere pressing against the skin. The bodily fluid is held within the region of the constriction and is also urged toward the incision by pressure generated by the constricting device.

In one embodiment, the constriction device comprises a deformable member positioned at the end of a support housing. The deformable member engages the skin as the device is initially pressed against the skin. As the device is further pressed against the skin, the member deforms such that the engaging surface grasps the skin and moves it inwardly toward the incision site. In this embodiment, the deformed member is preferably configured such that pressing the member against the skin automatically causes the member to deform in a manner that constricts the skin. In a preferred embodiment, the deformation of the member comprises non-permanent flexing of the member between the outer and inner positions. However, the invention also contemplates the use of a member that deforms in a manner that is permanent, or at least not fully reversible. The deforming member may be constructed from a variety of pliable, biocompatible materials suitable to produce such flexing or other deformation, including for example silicone, urethane, polyvinyl chloride, delrin, and various other natural and synthetic materials having the requisite physical properties.

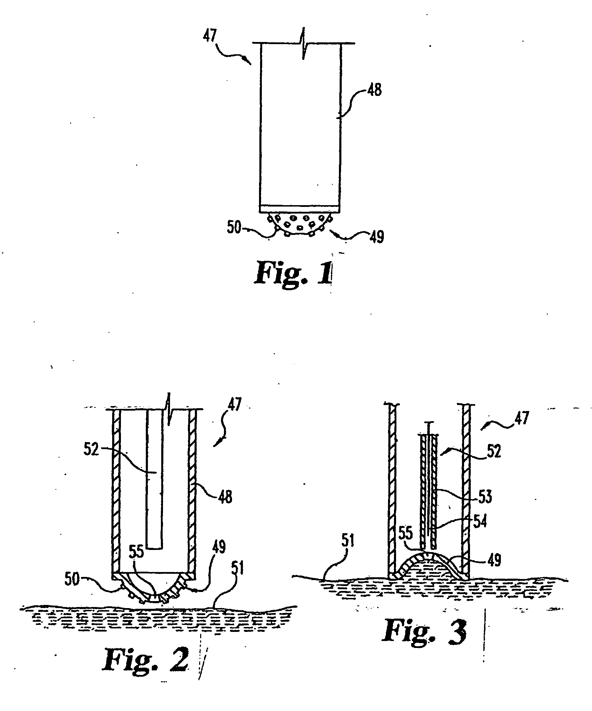

An embodiment within the purview of the present invention is shown in FIGS. 1-3. The device 47 comprises a housing 48 having a deformable expression member 49 secured to the distal end. The expression member may be formed from a variety of materials as previously described, and may be attached by any suitable means, including gluing or otherwise fastening the material to the housing. The expression member 49 is shown as having a generally rounded shape, although alternative shapes may be used. The member may include several projections 50 or other structures designed to engage the skin as described hereafter.

The expression member 49 is configured to have two stable positions with the member either extended or inverted, shown particularly in FIGS. 2 and 3, respectively. The member in the extended position is located adjacent the skin 51 in the area intended for fluid sampling. The device is then pressed against the skin and the member 49 inverts (FIG. 3), and in the process the skin is engaged and drawn into the cavity thereby formed in the expression member. This drawing in of the skin is facilitated by the engagement of the projections 50 with the skin. The member 49 thereby operates, in the manner previously discussed, to retain bodily fluid within the area and to apply pressure to the skin to facilitate the expression of fluid from an incision formed therein.

Once the bi-stable, dynamic bevel member 49 has been inverted (FIG. 3), the skin is lanced and the bodily fluid expressed from the incision. For example, the device 47 includes an annular capillary lancet system. The system includes a capillary tube 53 positioned within the housing 48 such that the end of the capillary will be proximate to the expression member 49 when such member is in the inverted position retaining the skin. The lancet 54 is extended beyond the end of the capillary tube and passes through a central opening 55 in the expression member to lance the skin. The lancet is then withdrawn and bodily fluid is expressed from the incision and through the opening 55. As the fluid sample grows it will make contact with the end of the capillary tube and will be drawn therein. Alternatively, the incision may be formed prior to applying the bi-stable member to the skin, but this is not preferred.

The opening 55 is sized to allow for the expression of the fluid from the incision. In one respect, the size of the opening is not critical, provided that it is large enough to permit the fluid to pass readily therethrough. In some instances, however, it may be desirable to provide an opening of a given, minimum dimension in order to further enhance the expression of the fluid. The opening may have any desired shape, but it is typically round and preferably has a minimum dimension of about 2 mm, and more preferably at least about 7 mm.

The outer surface of the constriction member 49 may be configured in a variety of ways to promote engagement of the skin. In a preferred embodiment, the member 49 includes outwardly-extending projections 50 that will contact and grasp the skin as the member inverts. The member may alternatively include other surface features to promote the engagement of the skin, including various surface projections or textures, or treatments such as coatings which stick to the skin.

The constriction devices of the present invention provide several advantages for the expression of a bodily fluid from an incision. As already described, the constriction of the skin maintains bodily fluid within the area of the incision site, and also applies a pressure to the skin that will tend to force fluid toward the incision. The constriction devices in certain embodiments also apply pressure to the skin in a manner which increases with the distance from the incision. For example, the drawing in of the skin by the bi-stable dynamic bevel will result in the skin being drawn the tightest in the area adjacent to the perimeter of the member 49, with less tension present toward the center. This provides a greater force at the perimeter to maintain the fluid therein, and to urge the fluid toward the center, and at the same time provides less tension toward the center in order to allow the fluid to move more freely toward and out of the incision. Correspondingly, the constriction devices in certain embodiments provide a pulling force at the center of the constricted space, thereby urging the incision open to facilitate the expression of bodily fluid from the incision.

The present invention also contemplates the use of members which are pressed against the skin, as distinguished from constricting the skin, to enhance the expression of bodily fluid from an incision.

It will be appreciated from the foregoing descriptions that the several forms of expression comprising the present invention are useful independently of the presence or type of incising, sampling or testing systems. In certain embodiments, however, the expression mechanisms and methods are combined with incising, sampling and/or testing systems. It will be appreciated by those skilled in the art that the function of the expression system is achieved independent of the incising and sampling systems, and therefore is useful with a variety of such systems as are known in the art. However, the expression systems are advantageously combined with incising and sampling systems in a single, integrated device. Because the expression is achieved essentially independently of these other systems, the expression system is readily adapted as an additional component of such devices. It will similarly be appreciated that the integrated device may also combine testing means to test desired constituents or characteristics of the fluid sample that has been acquired. Further, this integrated operation is available for all of the expression systems described herein. For example, the expression systems are useful in combination with a wide range of incising, sampling and testing systems, including those herein described in the description of the prior art and elsewhere, and the disclosures of such patents are hereby incorporated by reference.

As shown in the drawings, such an integrated device preferably operates such that the device does not have to be repositioned at any time during the process of incising, expressing, and/or sampling. More specifically, the device preferably carries incising, expressing, sampling and testing systems to perform a complete, integrated monitoring of the bodily fluid. In accordance with this approach, the device is moved against the skin and is maintained in this position while the incision is formed, and also while the resulting fluid droplet develops and is carried into the sampling device. The fluid is then analyzed by the test system and the result of the analysis is provided to the user. All of these actions therefore may be accomplished by a single, integrated unit, providing a simple, quick and reliable method for acquiring and testing a bodily fluid.

Moreover, the combination of the various systems in a single unit assures that the separate systems will be properly coordinated in use. The timing for the formation of the constricted pinch of skin and the lancing of the skin can be controlled automatically, or by manual operation by the user. The positions of the incision site and of the sampling capillary tube are predetermined to optimize the acquisition of the fluid formed at the incision site. This reduces the potential for a user to fail to successfully collect the fluid that is produced.

While the invention has been illustrated and described in detail in the drawings and foregoing description, the same is to be considered as illustrative and not restrictive in character, it being understood that only the preferred embodiment has been shown and described.

## Claims

1. A device (47) for expressing bodily fluid from an incision the skin, the incision being formed at an incision site, the device comprising:
a body defining a cavity; and
a bi-stable expression member (49) connected with said body adjacent to the interior cavity and including a portion having first and second stable conditions, said portion in the first stable condition being outwardly bulged relative to said body, said portion in the second stable condition being inverted and received within the cavity of said body, said portion including an exterior skin-engaging surface and defining an aperture (55) communicating between the exterior skin-engaging surface and the cavity of said body, the skin-engaging surface engaging the skin and drawing the skin into said member as said portion inverts from the first condition to the second condition.

2. The device of claim 1 in which said member is configured to automatically invert from the first condition to the second condition upon pressing said member against the skin.

3. The device of claim 1 in which said body comprises a cylindrical portion including a ring-shaped end surface, and in which said member comprises a semispherical member including a circular rim secured to the cylindrical end portion.

4. The device of claim 1 in which the exterior skin-engaging surface of said member includes a plurality of projections (50) for engaging the skin as said member inverts from the first condition to the second condition.

5. The device of claim 1 in which the aperture has a minimum dimension of at least about 2 mm.

6. The device of claim 5 in which the aperture is round and has a diameter of at least about 7 mm.

7. The device of claim 1 and which further includes an incising mechanism connected to said body and positioned to incise a person's skin at a location interior of said member.

8. The device of claim 7 in which said incising mechanism comprises a lancing device (54) positioned to lance the person's skin.

9. The device of claim 8 in which said lancing device comprises a lancet positioned and operable to incise the skin through the aperture in said member.

10. The device of claim 1 which further includes a sampling device (52) connected to said body.

11. The device of claim 1 and which further includes a test system connected to said body and in fluid communication with said sampling device.

12. The device of claim 1 and which further includes an incising mechanism connected to said body and positioned to incise a person's skin at a location interior of said member.

13. The device of claim 12 in which said incising mechanism comprises a lancing device positioned to lance the person's skin.

14. The device of claim 13 and which further includes a test system connected to said body and in fluid communication with said sampling device.

15. The device of claim 1 which said member is deformable.

16. The device of claim 15 in which said member is comprised of a material selected from the group consisting of silicone, urethane, polyvinyl chloride, and delrin.

## Patentansprüche

1. Vorrichtung (47) zum Herausdrücken von Körperflüssigkeit aus einem Einschnitt in der Haut, wobei der Einschnitt an einer Einschnittstelle geformt wird und die Vorrichtung Folgendes umfasst:
einen Körper, der einen Hohlraum definiert; und
ein bistabiles Ausdrückelement (49), das mit dem Körper neben dem inneren Hohlraum verbunden ist und einen Teil mit ersten und zweiten stabilen Zuständen aufweist, wobei der Teil im ersten stabilen Zustand relativ zum Körper nach außen gewölbt ist, wobei der Teil im zweiten stabilen Zustand nach innen gekehrt ist und im Hohlraum des Körpers aufgenommen wird, wobei der Teil eine äußere die Haut ergreifende Oberfläche aufweist und eine Öffnung (55) definiert, die die äußere, die Haut ergreifende Oberfläche mit dem Hohlraum des Körpers verbindet, wobei die die Haut ergreifende Oberfläche in die Haut eingreift und die Haut in das Element zieht, wenn der Teil von ersten Zustand in den zweiten Zustand geht.

2. Vorrichtung nach Anspruch 1, worin das Element so konfiguriert ist, dass es automatisch vom ersten Zustand in den zweiten Zustand geht, wenn das Element gegen die Haut gedrückt wird.

3. Vorrichtung nach Anspruch 1, worin der Körper einen zylindrischen Teil mit einer ringförmigen Endfläche aufweist und wobei das Element ein halbkreisförmiges Element umfasst, das einen am zylindrischen Endteil befestigten kreisförmigen Rand aufweist.

4. Vorrichtung nach Anspruch 1, worin die äußere die Haut ergreifende Oberfläche des Elements eine Vielzahl von Vorsprüngen (50) zum Eingriff in die Haut aufweist, wenn das Element vom ersten Zustand in den zweiten Zustand geht.

5. Vorrichtung nach Anspruch 1, worin die Öffnung eine Mindestabmessung von mindestens ungefähr 2 mm aufweist.

6. Vorrichtung nach Anspruch 5, worin die Öffnung rund ist und einen Durchmesser von mindestens ungefähr 7 mm aufweist.

7. Vorrichtung nach Anspruch 1, die ferner einen Einschneidemechanismus aufweist, der mit dem Körper verbunden ist und so positioniert ist, dass er an einer Stelle im Inneren des Elements die Haut einer Person einschneidet.

8. Vorrichtung nach Anspruch 7, worin der Einschneidemechanismus eine Lanzettenvorrichtung (54) aufweist, die so positioniert ist, dass sie in die Haut einer Person einsticht.

9. Vorrichtung nach Anspruch 8, worin die Lanzettenvorrichtung eine Lanzette aufweist, die so positioniert und bedienbar ist, dass sie die Haut durch die Öffnung im Element einschneidet.

10. Vorrichtung nach Anspruch 1, die ferner eine Entnahmevorrichtung (52) aufweist, die mit dem Körper verbunden ist.

11. Vorrichtung nach Anspruch 1, die ferner ein Testsystem aufweist, das mit dem Körper verbunden ist und mit der Entnahmevorrichtung in Flüssigkeitsverbindung steht.

12. Vorrichtung nach Anspruch 1, die ferner einen Einschneidemechanismus aufweist, der mit dem Körper verbunden ist und so positioniert ist, dass er an einer Stelle im Inneren des Elements die Haut einer Person einschneidet.

13. Vorrichtung nach Anspruch 12, worin der Einschneidemechanismus eine Lanzettenvorrichtung aufweist, die so positioniert ist, dass sie in die Haut einer Person einsticht.

14. Vorrichtung nach Anspruch 13, die ferner ein Testsystem aufweist, das mit dem Körper verbunden ist und mit der Entnahmevorrichtung in Flüssigkeitsverbindung steht.

15. Vorrichtung nach Anspruch 1, worin das Element verformbar ist.

16. Vorrichtung nach Anspruch 15, worin das Element aus einem Material besteht, das unter der aus Silikon, Urethan, Polyvinylchlorid und Delrin bestehenden Gruppe ausgewählt ist.

## Revendications

1. Dispositif (47) pour exprimer un fluide corporel à partir d'une incision de la peau, l'incision étant formée au niveau d'un site d'incision, le dispositif comprenant :
un corps définissant une cavité ; et
un élément d'expression bistable (49) relié audit corps adjacent à la cavité intérieure et incluant une partie ayant des premier et deuxième états stables, ladite partie dans le premier état stable étant extérieurement renflée par rapport audit corps, ladite partie dans le second état stable étant inversée et reçue à l'intérieur de la cavité dudit corps, ladite partie incluant une surface extérieure engageant la peau et définissant une ouverture (55) communiquant entre la surface extérieure engageant la peau et la cavité dudit corps, la surface engageant la peau engageant la peau et tirant la peau dans ledit élément comme ladite partie s'inverse du premier état au second état.

2. Dispositif selon la revendication 1, dans lequel ledit élément est configuré pour s'inverser automatiquement du premier état au second état à la pression dudit élément contre la peau.

3. Dispositif selon la revendication 1, dans lequel ledit corps comprend une partie cylindrique incluant une surface d'extrémité en forme d'anneau, et dans lequel ledit élément comprend un élément semi-sphérique incluant un rebord circulaire fixé à la partie d'extrémité cylindrique.

4. Dispositif selon la revendication 1, dans lequel la surface extérieure engageant la peau dudit élément inclut une pluralité de projections (50) pour engager la peau comme ledit élément s'inverse du premier état au second état.

5. Dispositif selon la revendication 1, dans lequel l'ouverture présente une dimension minimale d'au moins environ 2 mm.

6. Dispositif selon la revendication 5, dans lequel l'ouverture est ronde et présente un diamètre d'au moins environ 7 mm.

7. Dispositif selon la revendication 1, et qui inclut en outre un mécanisme d'incision relié audit corps et placé pour inciser la peau d'une personne au niveau d'un emplacement intérieur audit élément.

8. Dispositif selon la revendication 7, dans lequel ledit mécanisme d'incision comprend un dispositif de perçage (54) placé pour percer la peau de la personne.

9. Dispositif selon la revendication 8, dans lequel ledit dispositif de perçage comprend une lancette placée et pouvant être mise en action pour inciser la peau à travers l'ouverture dans ledit élément.

10. Dispositif selon la revendication 1, qui inclut en outre un dispositif d'échantillonnage (52) relié audit corps.

11. Dispositif selon la revendication 1, et qui inclut en outre un système d'essai relié audit corps et en communication fluide avec ledit dispositif d'échantillonnage.

12. Dispositif selon la revendication 1, et qui inclut en outre un mécanisme d'incision relié audit corps et placé pour inciser la peau d'une personne au niveau d'un emplacement intérieur audit élément.

13. Dispositif selon la revendication 12, dans lequel ledit mécanisme d'incision comprend un dispositif de perçage placé pour percer la peau d'une personne.

14. Dispositif selon la revendication 13, et qui inclut en outre un système d'essai relié audit élément et en communication fluide avec ledit dispositif d'échantillonnage.

15. Dispositif selon la revendication 1, dont ledit élément est déformable.

16. Dispositif selon la revendication 15, dans lequel ledit élément est composé d'un matériau choisi d ans le groupe constitué de la silicone, l'uréthane, le poly(chlorure de vinyle), et le delrine.
